# EUROPEAN PATENT APPLICATION

(11) **EP 0 751 149 A2**
(43) Date of publication of application: **02.01.1997**
(21) Application number: 96201385.0
(22) Date of filing: 28.06.1990
(51) Int. Cl.: C07K 14/46, C07K 7/08, A61K 38/17, A61K 38/10

(54) **Phenylalanine substitution analogues of magainin peptides**

(30) Priority: 07.07.1989 US 376754
(62) Divisional of application: 90909538.2
(71) Applicant: THE SCRIPPS RESEARCH INSTITUTE, La Jolla, CA 92037 (US)
(72) Inventor: Houghten, Richard A., Solana Beach, CA 92075 (US); Cuervo, Julio H., Cardiff, CA 92001 (US)
(74) Representative: Fisher, Adrian John

(57) **Abstract**

A compound comprising a Magainin I or Magainin II peptide wherein at least one amino acid residue is substituted with a phenylalanine residue. In addition to such substitutions, at least one of amino acid residues 15 to 23 may be deleted.

## Description

This invention relates to a class of biologically active peptides known as magainins. More particularly, this invention relates to analogues of magainin peptides wherein at least one amino acid residue in the peptide has been substituted with another amino acid residue, with said analogues being commonly referred to as "substitution analogues".

H-C Chen *et al*, FEBS Letters, 236 (2), 462-466 (1988) disclose a limited range of substitution analogues of Magainin II (mostly involving alanine substitutions at residues 8, 13 and 18), with improved antimicrobial activity. J.H. Cuervo *et al*, Peptide Research, 1 (2), 81-86 (1988) disclose amino acid omission analogues of Magainin I and Magainin II.

In accordance with the present invention, there is provided a compound comprising an analogue of Magainin I peptide or Magainin II peptide. The Magainin I or Magainin II peptide is in an amide or carboxy terminated form. Magainin I is represented by the following structural formula using the single letter amino acid code and the numbers below each amino acid residues refer to the position of the residue in the peptide. 
G I G K F L H S A G K F G K A F V G E I M K S
1 2 3 4 5 6 7 8 9 10 11 12 13 14 15 16 17 18 19 20 21 22 23
 Magainin II is represented by the following structural formula using the single letter amino acid code and the numbers below each amino acid residue refer to the position of the residue in the peptide:

G I G K F L H S A K K F G K A F V G E I M N S
1 2 3 4 5 6 7 8 9 10 11 12 13 14 15 16 17 18 19 20 21 22 23

In the analogues of the present invention, any of amino acid residues 15-23 of Magainin I peptide or Magainin II peptide is optionally omitted, and (if amino acid residue 19 is omitted) amino acid residues 1-4 or 3, 5 and 6 may be omitted. One to five of the remaining amino acid residues of said Magainin peptide is substituted by another amino acid residue, at least one of amino acid residues 13, 15 and 18 being substituted by phenylalanine and any further substitutions being as shown in the following table, provided that residue 18 may be substituted by Phe only if one of residues 15 to 17 or 19 to 23 is omitted.

| Residue No. | Substituent |
|---|---|
| 1 | Lys, Ala |
| 2 | Lys, Ala, D-Ile, Arg, Leu, Val, His, Met |
| 3 | Lys, Ala, Trp, Arg, His |
| 4 | D-Lys, Ala, Arg, His |
| 5 | D-Phe, Ala, Lys, Trp, Leu, Ile, Val, Met |
| 6 | D-Leu, Lys, Ala, Ile, Val, Met |
| 7 | Lys, D-His, Ala, Arg, Ile, Val, Met |
| 8 | Ala, Lys, D-Ser, Trp, Met, Ile, Arg, His, Thr, Leu, Val |
| 9 | Lys, D-Ala, Trp, Arg, His, Leu, Ile, Val |
| 10 | D-Lys, Lys, Ala, Trp, Arg, His, Leu, Ile, Val |
| 11 | D-Lys, Arg, His |
| 12 | D-Phe, Lys, Trp, Arg, His |
| 13 | Ala, Lys, Trp, Met, Arg, His, Phe, Leu, Ile, Val |
| 14 | Ala, D-Lys, Arg, His |
| 15 | Lys, Trp, D-Ala, Arg, His, Phe |
| 16 | Ala, Lys, D-Phe, Ile, Val, Met, Leu |
| 17 | Ala, Lys, D-Val, Trp, Arg, His, Met, Leu |
| 18 | Ala, Lys, Trp, Arg, His, Leu, Met, Phe |
| 19 | Ala, Lys, D-Glu, Gly, Arg, His, Leu, Ile, Phe, Asn |
| 20 | D-Ile, Ala, Lys |
| 21 | Lys, Pro, Ala, His, Leu, Arg, Ile, Phe |
| 22 | Lys, Ala, D-Asn, Gln, Arg, His |
| 23 | D-Ser, Lys, Ala, Thr, Gly, Leu, Ile, Gln, Asn |

It is to be understood that for purposes of the present invention, the tryptophan residues may be protected with a formyl group or be unprotected. The phenylalanine residues, whether such residue is present in its normal position, or employed as a substitution residue, may be a normal phenylalanine residue or an iodinated phenylalanine residue.

In a preferred embodiment, the peptide is a Magainin II peptide, and the substitution analogue wherein amino acid 19 is deleted is selected from the class consisting of the following substitution analogues:
GIGKFLHSAKKFGKAFVFIMNS*, wherein
S* is D-Serine;
GIGKFLHSAKKFFKAFVFIMNS;
GIGKFLKSAKKFGKAFVFIMNS;
GIGKFLHKAKKFAKAFVFIMNS;
GIGKFLKSAKKFAKAFVFIMNS;
GIGKFLHKAKKFAKAFVFIMNK; and
GIGKFLKKAKKFGKAFVFIMKK.

Preliminary studies indicate that these preferred analogues possess low hemolytic activity.

Applicants have found that when employing the substitution analogues of Magainin I or Magainin II as hereinabove described, such peptides display biological activity equal to or greater than the parent Magainin I or Magainin II peptide. Such peptides are referred to as "successful substitution analogues".

The use of these compounds which comprise a substitution analogue of Magainin I or Magainin II peptide, in accordance with the present invention, is effective as an antibiotic, and can be employed to inhibit, prevent or destroy the growth or proliferation of microbes, such as bacteria, fungi viruses or the like. Similarly, such compounds can be employed as an anti-viral composition to inhibit, prevent or destroy the growth or proliferation of viruses.

As used herein, the term "substitution analogue" includes magainin peptides in which at least one amino acid residue of the peptide structure has been substituted with a different amino acid residue, as well as magainin peptides in which, in addition to the above-mentioned substitution(s), also have had at least one of amino acid residues 15-23 omitted from the peptide sequence. The term also includes magainin peptides having D-amino acid or iodinated phenylalanine residues which have been substituted into the peptide sequence.

Such compounds can also be employed as a spermicide to inhibit, prevent or destroy the motility of sperm.

Such compounds can also be employed as anti-tumor agents to inhibit the growth of or destroy tumors.

The compounds have a broad range of potent antibiotic activity against a plurality of microorganisms, including Gram-positive and Gram-negative bacteria, fungi, protozoa and the like. Such compounds can be employed for treating or controlling microbial infection caused by organisms which are sensitive to such compounds.

The compounds can also be used as preservatives or sterilants for materials susceptible to microbial contamination. In vitro activity against bacteria is exemplified hereinafter in Examples 3 and 4.

In general, a substitution analogue of the Magainin I or Magainin II peptide is administered in a dosage of from about 1 mg to about 500 mg per kilogram of body weight, when administered systemically. When administered topically, the peptide is used in a concentration of from about 0.5% to about 5%.

The compounds comprising the substitution analogues of Magainin I or Magainin II, in accordance with the present invention, can be employed for treating a wide variety of hosts. In accordance with a preferred embodiment, a host can be an animal, and such animal can be human or non-human animal.

The compounds comprising the substitution analogues of Magainin I or Magainin II can be employed in a wide variety of pharmaceutical compositions in combination with a non-toxic pharmaceutical carrier or vehicle such as a filler, non-toxic buffer, or physiological saline solution. Such pharmaceutical compositions can be used topically or systemically and can be in any suitable form such as a liquid, solid, semi-solid, injectable solutions, tablet, ointment, lotion, paste, capsule or the like. The compounds comprising the substitution analogues of Magainin I or Magainin II can also be used in combination with adjuvants, protease inhibitors, or compatible drugs where such a combination is seen to be desirable or advantageous in controlling infection caused by harmful microorganisms including protozoa, viruses, and the like.

The compounds comprising the substitution analogues of Magainin I or Magainin II of the present invention can be administered to a host; in particular an animal, in an effective antibiotic and/or anti-tumor and/or anti-viral and/or anti-microbial and/or a spermicidal amount.

Magainin I and Magainin II, as well as the substitution analogues of the Magainin I and Magainin II peptides of the present invention, (both amide- and carboxy-terminated Magainin I and Magainin II forms) can be synthesized by any convenient method of peptide synthesis as are well-known to skilled workers. Solid phase synthesis methods are particularly preferred.

The peptides described herein were prepared by the method of simultaneous multiple peptide synthesis (SMPS). This method is described in detail in Houghten, R.A., "General Method for the Rapid Solid-Phase Synthesis of Large Numbers of Peptides; Specificity of Antigen-Antibody Interaction at the Level of Individual Amino Acids," Proc. Natl. Acad. Sci., U.S.A. Vol. 82, pgs 5131-5135 (1985), and in Houghten, R.A., et al. "Simultaneous Multiple Peptide Synthesis; The Rapid Preparation of Large Numbers of Discrete Peptides for Biological, Immunological, and Methodological Studies," Peptide Chemistry, pgs. 295-298 (1987).

For the purposes of the following examples, substitution analogues of Magainin I and Magainin II were prepared wherein various amino acid residues were substituted and/or other amino acid residue(s) were deleted.

For the purposes of comparison, a complete Magainin I or Magainin II peptide can also be prepared by the SMPS method. It is also contemplated within the scope of the present invention that substitution analogues which have one or more amino acid residue(s) omitted from the Magainin I or Magainin II structure can also be prepared by the SMPS method.

The invention will now be described with respect to the following examples. However, the scope of the present invention is not intended to be limited thereby.

### Example 1 - Peptide Synthesis

Peptide synthesis of Magainin I and the substitution analogues of Magainin I, was accomplished by using the strategy of simultaneous multiple peptide synthesis. All solvents and reagents were of analytical grade and were used without further purification. Standard N-t-Boc-protected amino acids were employed in the synthesis. The side chain functionalities used were benzyl (Ser, Glu), 2-Cl-Z (Lys)(Z=benzyloxycarbonyl), N^{im}- DNP (His), and sulfoxide (Met). Peptide synthesis was performed beginning with 100 mg of either Boc-amino acids-Pam resin to produce C-terminal carboxyl peptide (PAM purchased from Applied Biosystems, substitution 0.56 meq/gm is an amino acyl-4-[oxymethyl] phenylacetic acid derivative of amino polystyrene) or methylbenzhydrylamine (MBHA) resin (substitution-0.65 meq/gm) per resin packet to produce C-terminal amide peptide.

After synthesis, completely protected peptide resins were treated three times with 0.5 M thiophenol in DMF to remove the N^{im}- dinitrophenyl group from Histidine. The final Boc-group was removed with TFA to avoid t-butylation of methionyl residues during final HF treatment. Cleavage was performed using the Low-High HF procedure. Tam, et al. J. Am. Chem. Soc, Vol. 105, P. 6442 (1983). For peptides synthesized on Pam resin, the low-HF was carried out without removing the resin from the packet, using a multiple vessel HF apparatus for 2 hrs. at O°C. For peptides prepared using MBHA resin, the low HF procedure was performed in a common reaction vessel for 2 hrs. at O°C. For Pam resin peptides, the low-HF mixture was evacuated from the 24 individual reaction vessels by a water aspirator followed by a mechanical pump. The low-HF reaction vessel containing the bags with MBHA resin was emptied of the low-HF mixture by pouring off the liquid into a waste container. The bags were washed immediately with cold ether followed by alternating washes of CH₂Cl₂, DMF, CH₂Cl₂, IPA, CH₂Cl₂. The packets were then dried and put into individual tubes of the 24 vessel HF apparatus with 0.7ml of anisole as scavenger. The high-HF was performed by condensing dry hydrogen fluoride at -70°C. The reaction took place at -10°C for 1h. and -5°C - 0°C for the last 30 min. HF was evaporated using a strong flow of nitrogen. Finally, residual carbonium ion scavengers were removed by washing with dry ether.

The crude peptide were subsequently extracted with 10% acetic acid and subjected to RP-HPLC on an analytical reverse phase column (Vidac ODS 25 cm x 4.6 mm), using a Beckman-Altek model 421 HPLC system and two model 110A pumps. The solvent system was composed of buffer A, 0.05% TFA/H₂O, and buffer B, 0.05% TFA/CH₃CN with a flow rate of 1.0 ml/min. The peptides were detected at 215 nm using a Hitachi 100-20 spectrophotometer.

Purification of the peptides was accomplished by reverse-phase HPLC on a Vidac C₁₈ (22 mm x 25 cm), 10 µm packing column with an eluting gradient composed of CH₃CN and 0.05% TFA. Amino acid analysis was carried out on a Beckman 6300 analyzer following hydrolysis of the peptides in constant (boiling) 6 N HCl at 100°C for 24 hr., and such analysis was within ± 10% of theory.

### Example 2 - Antimicrobial Assays

Antimicrobial assays were carried out in 96-well tissue culture plates. Each well was incubated with a given microorganism (Escherichia coli, Staphylococcus epidermidis or Pseudomonas aeruginosa) suspended in TEB medium. Upon the addition of Magainin I or its substitution analogues, (dissolved in 1 x PBS, pH 7.0) each well contained a final cell density of 1.0 x 10⁶ colony forming units (CFU)/ml. The final peptide concentrations used were 100 µg/ml, 75.0 µg/ml, 50.0 µg/ml, 25 µg/ml, 12.5 µg/ml, 10 µg/ml, 5 µg/ml, 2.5 µg/ml, and 1.25 µg/ml.

Addition of peptides to the wells was defined as time zero. At six hours, the plates were placed in a Titertek Multiskan apparatus and the O.D.₆₂₀ determined. The plates as well as the initial inoculum were incubated at 37°C.

Five wells per plate contained media alone, while five others contained medium plus cells. These controls were used to eliminate the possibility of media contamination while providing a measure of uninhibited growth of the microorganisms.

The degree of peptide activity was determined by comparing the substitution analogues with uninhibited growth of the control cells over a six-hour period. The effective growth inhibition of the substitution analogues is listed in the examples and tables below.

### Example 3 - Magainin II Analogues with Glutamic Acid Deletions

In this example, analogues of Magainin II were synthesized, said analogues having amino acid residue 19 (glutamic acid) deleted from the peptide structure, and wherein one or more of the other amino acid residues was substituted with an amino acid residue different from the normal amino acid rseidue. Analogues of the following structures were then synthesized according to the method described in Example 1.

| Analogue | Structure |
|---|---|
| 1 | GILKFLHSAKKFFKAFVKILNS |
| 2 | GIGKFLHSAKKFFKAFVFILNS |
| 3 | GIGKFLHSAKKFGKAFVFIMNS* |
| 4 | GIGKFLHSAKKFAKAFVFIMNS |
| 5 | GIGKFLHSAKKFFKAFVFIMNS |
| 6 | GIGKFLKSAKKFGKAFVFIMNS |
| 7 | GIGKFLHKAKKFAKAFVFIMNS |
| 8 | GIGKFLKSAKKFAKAFVFIMNS |
| 9 | GIGKFLHKAKKFFKAFVFIMNS |
| 10 | GIGKFLKSAKKFFKAFVFIMNS |
| 11 | GIGKFLHKAKKFAKAFVFIMNK |
| 12 | GIGKFLKSAKKFAKFFVFIMNK |
| 13 | GIGKFLKKAKKFGKAFVFIMKK |

| | |
|---|---|
| *D-Serine | |

Each of the peptides were then tested as hereinabove described for % effective growth inhibition of E. coli, P. aeruginosa, and S. epidermidis in µg/ml. The % effective growth inhibition of each of these analogues is given in Table I below.

**TABLE I**

| % Effective Growth Inhibition, Concentration in µg/ml | | | |
|---|---|---|---|
| Analogue | E. coli | P. aeruginosa | S. epidermidis |
| 1 | 100 at 2.5 | 100 at 2.5 | 100 at 5 |
| 2 | 100 at 10 | 100 at 5 | 100 at 5 |
| 3 | 100 at 5 | 100 at 5 | 100 at 5 |
| 4 | 100 at 5 | 100 at 2.5 | 100 at 5 |
| 5 | 100 at 5 | 100 at 10 | 100 at 5 |
| 6 | 100 at 5 | 100 at 5 | 100 at 5 |
| 7 | 100 at 1.25 | 100 at 2.5 | 100 at 1.25 |
| 8 | 100 at 2.5 | 100 at 1.25 | 100 at 2.5 |
| 9 | 100 at 10 | 100 at 2.5 | 100 at 2.5 |
| 10 | 100 at 5 | 100 at 5 | 100 at 1.25 |
| 11 | 100 at 1.25 | 100 at 1.25 | 100 at 1.25 |
| 12 | 100 at 1.25 | 100 at 5 | 100 at 2.5 |
| 13 | 100 at 2.5 | 100 at 2.5 | 100 at 2.5 |

### Example 4

An analogue of Magainin II was synthesized wherein amino acid residues 1 to 4 and 19 were deleted, and amino acid residue 18 was substituted with phenylalanine. The analogue was of the following structure:
FLHSAKKFGKAFVFIMNS
The analogue was then tested for % effective growth inibition of E.coli, P.aeruginosa, and S. epidermidis. The % effective growth inhibition was 100% at 25 µg/ml for E. coli, 100% at 25 µg/ml for P. aeruginosa and 100% at 5 µg/ml for S. epidermidis.

Numerous modifications and variations of the present invention are possible in light of the above teachings, and, therefore, within the scope of the accompanying claims, the invention may be practised other than as particularly described.
*D-Serine.

## Claims

1. A compound having antimicrobial, antiviral or antitumor activity, comprising an analogue of Magainin I peptide or Magainin II peptide in an amide- or carboxy- terminated form, wherein Magainin I is represented by the following structural formula using the single letter amino acid code and the numbers below each residue refer to the position of the residue in the peptide: 
G I G K F L H S A G K F G K A F V G E I M K S
1 2 3 4 5 6 7 8 9 10 11 12 13 14 15 16 17 18 19 20 21 22 23
 and Magainin II is represented by the following structural formula using the single letter amino acid code and the numbers below each amino acid residue refer to the position of the residue in the peptide: 
G I G K F L H S A K K F G K A F V G E I M N S
1 2 3 4 5 6 7 8 9 10 11 12 13 14 15 16 17 18 19 20 21 22 23
 said analogue being characterised in that any of amino acid residues 15-23 of Magainin I peptide or Magainin II peptide is optionally omitted (and, if amino acid residue 19 is omitted, amino acid residue 1-4 or 3, 5 and 6 may also be omitted), and one to five of the remaining amino acid residues of said Magainin peptide is substituted by another amino acid residue, at least one of amino acid residues 13, 15 and 18 being substituted by phenylalanine and any further substitutions being as shown in the following table, provided that residue 18 may be substituted by Phe only if one of residues 15 to 17 or 19 to 23 is omitted:
| Residue No. | Substituent |
|---|---|
| 1 | Lys, Ala |
| 2 | Lys, Ala, D-Ile, Arg, Leu, Val, His, Met |
| 3 | Lys, Ala, Trp, Arg, His |
| 4 | D-Lys, Ala, Arg, His |
| 5 | D-Phe, Ala, Lys, Trp, Leu, Ile, Val, Met |
| 6 | D-Leu, Lys, Ala, Ile, Val, Met |
| 7 | Lys, D-His, Ala, Arg, Ile, Val, Met |
| 8 | Ala, Lys, D-Ser, Trp, Met, Ile, Arg, His, Thr, Leu, Val |
| 9 | Lys, D-Ala, Trp, Arg, His, Leu, Ile, Val |
| 10 | D-Lys, Lys, Ala, Trp, Arg, His, Leu, Ile, Val |
| 11 | D-Lys, Arg, His |
| 12 | D-Phe, Lys, Trp, Arg, His |
| 13 | Ala, Lys, Trp, Met, Arg, His, Phe, Leu, Ile, Val |
| 14 | Ala, D-Lys, Arg, His |
| 15 | Lys, Trp, D-Ala, Arg, His, Phe |
| 16 | Ala, Lys, D-Phe, Ile, Val, Met, Leu |
| 17 | Ala, Lys, D-Val, Trp, Arg, His, Met, Leu |
| 18 | Ala, Lys, Trp, Arg, His, Leu, Met, Phe |
| 19 | Ala, Lys, D-Glu, Gly, Arg, His, Leu Ile, Phe, Asn |
| 20 | D-Ile, Ala, Lys |
| 21 | Lys, Pro, Ala, His, Leu, Arg, Ile, Phe |
| 22 | Lys, Ala, D-Asn, Gln, Arg, His |
| 23 | D-Ser, Lys, Ala, Thr, Gly, Leu, Ile, Gln, Asn |

2. A compound according to claim 1, wherein at least one of amino acid residues 15-23 is omitted.

3. A compound according to claim 2 wherein amino acid residue 19 is omitted.

4. A compound according to any of claims 1 to 3, which is an analogue of Magainin II peptide.

5. The compound of claim 1 wherein said analogue of Magainin I peptide or Magainin II peptide is selected from:
GILKFLHSAKKFFKAFVKILNS;
GIGKFLHSAKKFFKAFVFILNS;
GIGKFLHSAKKFGKAFVFIMNS*;
GIGKFLHSAKKFAKAFVFIMNS;
GIGKFLHSAKKFFKAFVFIMNS;
GIGKFLKSAKKFGKAFVFIMNS;
GIGKFLHKAKKFAKAFVFIMNS;
GIGKFLKSAKKFAKAFVFIMNS;
GIGKFLHKAKKFFKAFVFIMNS;
GIGKFLKSAKKFFKAFVFIMNS;
GIGKFLHKAKKFAKAFVFIMNK;
GIGKFLKSAKKFAKFFVFIMNK;
GIGKFLKKAKKFGKAFVFIMKK; and
FLHSAKKFGKAFVFIMNS

6. A process comprising:
administering to a host an effective spermicidal amount of the compound of any of claims 1 to 5.

7. The use of a compound according to any of claims 1 to 5 in the manufacture of a medicament for treating microbial infections.

8. The use of a compound according to any of claims 1 to 5 in the manufacture of a medicament for treating viral infections.

9. The use of a compound according to any of claims 1 to 5 in the manufacture of a medicament for treating tumors.
